(19) Europäisches Patentamt

European Patent Office

Office européen des brevets

(11) **EP 0 804 234 B1**

(12) **FASCICULE DE BREVET EUROPEEN**

(45) Date de publication et mention
de la délivrance du brevet:
**02.06.1999 Bulletin 1999/22**

(21) Numéro de dépôt: **95942006.8**

(22) Date de dépôt: **21.12.1995**

(51) Int. Cl.[6]: **A61K 39/39**

(86) Numéro de dépôt international:
**PCT/BE95/00118**

(87) Numéro de publication internationale:
**WO 96/20007 (04.07.1996 Gazette 1996/30)**

(54) **ADJUVANT POUR VACCINS**

ADJUVANS FÜR IMPSTOFFE

VACCINE ADJUVANT

(84) Etats contractants désignés:
**AT BE CH DE DK ES FR GB GR IE IT LI LU NL PT
SE**
Etats d'extension désignés:
**LT LV SI**

(30) Priorité: **27.12.1994 BE 9401173**

(43) Date de publication de la demande:
**05.11.1997 Bulletin 1997/45**

(73) Titulaire: **DIMMINACO AG
8134 Adliswil (CH)**

(72) Inventeurs:
• **HILGERS, Luuk
NL-3583 HE Utrecht (NL)**
• **STREBELLE, Michel
B-1150 Bruxelles (BE)**

(74) Mandataire:
**Talbott, Dawn Jacqueline
Wyeth Laboratories,
Patent Department,
Huntercombe Lane South,
Taplow
Maidenhead, Berkshire SL6 0PH (GB)**

(56) Documents cités:
**EP-A- 0 273 512          WO-A-92/04915
US-A- 3 790 665**

• **INFECT. IMMUN. (1978), 19(2), 667-75 ,
KREUTER, JOERG ET AL 'Mode of action of
immunological adjuvants: some
physicochemical factors influencing the
effectivity of polyacrylic adjuvants'**
• **EUROPEAN JOURNAL OF IMMUNOLOGY, vol. 1,
pages 335-340, DIAMANSTEIN T. 'Stimulation of
humoral antibody formation by polyanions' cited
in the application**
• **VACCINE, vol. 8, no. 6, December 1990 pages
573-576, XP 000168477 TETSUYA OKA ET AL
'INFLUENZA VACCINE: ENHANCEMENT OF
IMMUNE RESPONSE BY APPLICATION OF
CARBOXY-VINYLPOLYMER'**
• **INFECTION AND IMMUNITY, vol. 13, no. 1,
January 1976 WASHINGTON US, pages 204-210,
KREUTER J. ET AL. 'New Adjuvants on a
Polymethacrylate Base'**
• **JOURNAL OF IMMUNOLOGY, vol. 133, no. 6,
December 1984 BALTIMORE US, pages 3167-
3175, HUNTER R.L. ET AL. 'THE ADJUVANT
ACTIVITY OF NONIONIC BLOCK POLYMER
SURFACTANTS'**

**Description**

La présente invention a pour objet de nouveaux adjuvants pour vaccins.

Un antigène est défini comme étant une substance étrangère, qui, lorsqu'on l'administre par exemple par voie parentérale, induit une réponse immunitaire, entre autres la production d'anticorps. Les anticorps sont des substances contenues dans le sang et autres fluides du corps ainsi que dans les tissus, qui se lient à l'antigène pour le rendre inoffensif. Les anticorps constituent un des mécanismes naturels de défense du corps. Ils sont hautement spécifiques et peuvent tuer, lier ou rendre inoffensif l'antigène qui a induit leur formation.

L'antigène, en contact avec le système immunitaire, active donc une serie complexe d'interactions cellulaires dont le but est d'éliminer l'antigène et/ou de rétablir l'équilibre précédent.

Deux des aspects caractéristiques des antigènes sont leur immunogénicité, c'est-à-dire leur capacité d'induire une réponse immunitaire _in vivo_ (entre autres la formation d'anticorps spécifiques) et leur antigénicité, c'est-à-dire leur capacité d'être reconnus spécifiquement par les anticorps dont les antigènes sont à l'origine.

Il est connu qu'il est possible de stimuler la réponse immunitaire délibérément en administrant un antigène spécifique au moyen d'un vaccin. Cette procédure permet de développer dans l'organisme un état de mémoire immunitaire qui assure une réponse plus rapide et plus efficace de l'organisme lors d'un contact ultérieur avec l'antigène.

Cependant, quelques antigènes ne possèdent qu'une faible immunogénicité et induisent une réponse immunitaire insuffisante pour procurer à l'organisme une protection efficace.

L'immunogénicité d'un antigène peut être accrue en l'administrant en mélange avec des substances, appelées adjuvants, qui augmentent la réponse contre l'antigène soit en agissant directement sur le système immunologique soit en modifiant les caractéristiques pharmacocinétiques de l'antigène et en augmentant ainsi le temps d'interaction de ce dernier avec le système immunitaire.

Les adjuvants les plus répandus sont d'une part, l'adjuvant de Freund, une émulsion comprenant des mycobactéries mortes en une solution saline dans une huile minérale et d'autre part, l'adjuvant de Freund incomplet qui ne comprend pas de mycobactéries.

Ces adjuvants sont capables soit d'augmenter l'intensité de la réponse immunitaire à l'antigène soit de produire une activation aspécifique du système immunitaire.

Cependant l'utilisation de ces adjuvants comporte des inconvénients tels que la formation d'irritations ou d'abcès au point d'injection. De plus, pour que ces adjuvants soient efficaces, la concentration à utiliser doit être supérieure à 50 % du volume injecté, ce qui limite la charge utile d'antigènes que l'on peut injecter en une dose.

La grande viscosité de ces adjuvants classiques à base d'huile et d'eau rend leur utilisation peu pratique car ils sont difficiles à introduire dans les seringues et à injecter dans les animaux.

Un autre type d'adjuvant décrit, comprend une solution d'acides polyacryliques [Diamanstein et al., Z. Klin. Chem. Klin. Biochem. 8,632-636 (1970) and Diamanstein et al., Eur. J. Immunol. 1,335-339 (1971)]. L'avantage de ce type d'adjuvant est qu'il est moins visqueux que les adjuvants classiques à base d'huile minérale et d'eau. Il peut donc être manipulé et injecté plus aisément. Cependant l'efficacité de ces adjuvants n'est pas comparable à celle des adjuvants à base d'eau dans l'huile minérale (W/O).

Vaccine, 8(6), 1990, 573-6 décrit d'utilisation d'un polymère carboxyvinylique (CVP) comme adjuvant dans des vaccins antigrippaux.

Le but de la présente invention est de proposer un adjuvant pour vaccins qui soit efficace en concentration faible et sans huile minérale.

Ce but est atteint par un adjuvant pour vaccins comprenant une solution aqueuse de polymères ayant des unités constitutionnelles répétitives anioniques et des unités contitutionnelles répétitives hydrophobes.

Par unités constitutionnelles répétitives anioniques, on entend désigner aux fins de la présente invention des unités monomériques, constitutives du polymère, qui contiennent des groupements capables de se dissocier dans l'eau en formant des anions.

Des exemples de telles unités monomériques utiles dans la présente invention pour former les unités constitutionnelles répétitives anioniques sont (choisies parmi) les acides acrylique, méthacrylique, maléique, fumarique, éthylènesulfonique, vinylsulfurique, styrènesulfonique, vinylphénylsulfurique, 2-méthacryloyloxy éthanesulfonique, 3-méthacryloyloxy-2-hydroxypropanesulfonique, 2-acryl-2-méthylpropanesulfonique, 3-acrylamido-3-méthylbutanoïque, 3-méthacrylamido-3-méthylbutanoïque, vinylphosphorique, 4-vinylbenzoïque, 3-vinyloxypropane-1-sulfonique et N-vinylsuccimidique.

De préférence, les unités monomériques de ce type sont choisies parmi les acides acrylique, méthacrylique, maléique, fumarique, éthylènesulfonique, vinylsulfurique et styrènesulfonique.

De manière préférée, les unités monomériques de ce type sont choisies parmi les acides acrylique, méthacrylique, maléique et fumarique.

De manière particulièrement préférée, les unités monomériques de ce type sont des unités d'acide acrylique.

Par unités constitutionnelles répétitives hydrophohes, on entend désigner aux fins de la présente invention des

2

unités monomériques, constitutives du polymère, qui contiennent exclusivement des groupements hydrophobes encore appelés lipophiles et qui ne se dissocient pas dans l'eau.

Des exemples de telles unités monomériques utiles dans la présente invention pour former les unités constitutionnelles répétitives hydrophobes sont (choisies parmi) les esters alkyliques, cycloalkyliques et hydroxyalkyliques des acides mentionnés ci-dessus (acides acrylique, méthacrylique, maléique, fumarique, éthylènesulfonique, vinylsulfurique, styrènesulfonique, vinylphénylsulfurique, 3-méthacryloyloxy-2-hydroxypropanesulfonique, 2-méthacryloyloxy éthanesulfonique, 2-acryl-2-méthylpropanesulfonique, 3-acrylamido-3-méthylbutanoïque, 3-méthacrylamido-3-méthylbutanoïque, vinylphosphorique, 4-vinylbenzoïque, 3-vinyloxypropane-1-sulfonique ou N-vinylsuccimidique), et les éthers (par exemple, méthoxyméthyl, éthoxyéthyl, allyloxyméthyl, 2-éthoxyéthoxyméthyl, benzyloxyméthyl, cyclohexylométhyl, 1-éthoxyéthyl, 2-éthoxyéthyl, 2-butoxyéthyl, méthoxyméthoxyéthyl, méthoxyéthoxyéthyl, 1-butoxypropyl, 1-éthoxybutyl, tétrahydrofurfuryl ou furfuryl).

De préférence, les unités monomériques de ce type sont choisies parmi les esters alkyliques des acides acrylique, méthacrylique, maléique, fumarique, éthylenesulfonique, vinylsulfurique ou styrènesulfonique.

De manière préférée, les unités monomériques de ce type sont choisies parmi les esters alkyliques des acides acrylique, méthacrylique, maléique ou fumarique dont le groupe alkyle contient de 4 à 8 atomes de carbone,

De manière particulièrement préférée les unités monomériques de ce type sont les esters alkyliques linéaires d'acide acrylique dont le groupe alkyle contient de 4 à 8 atomes de carbone.

De manière particulièrement préférée, les adjuvants utilisés selon la présente invention sont les solutions aqueuses de polymères dont les unités monomériques utiles pour former les unités constitutionelles répétitives anioniques sont constituées d'acide acrylique et dont les unités monomériques utiles pour former les unités constitutionnelles répétitives hydrophobes sont choisies parmi les esters alkyliques linéaires d'acide acrylique dont le groupe alkyle contient de 4 à 8 atomes de carbone.

La réponse humorale aux vaccins comprenant une solution aqueuse de polymères ayant des unités constitutionnelles répétitives anioniques et des unités constitutionnelles répétitives hydrophobes est supérieure à la réponse induite par des polymères ayant exclusivement des unités constitutionnelles répétitives anioniques, tels que, par exemple, les acides polyacryliques.

En effet, l'efficacité des adjuvants selon la présente invention est comparable à celle des adjuvants classiques à base d'eau dans l'huile minérale tandis que leur toxicité est, en général, nettement inférieure.

Les adjuvants selon la présente invention ne posent pas de problèmes d'instabilité comme les adjuvants classiques à base d'émulsion d'huile dans l'eau (O/W) ou d'eau dans l'huile (W/O) car ceux-ci sont toujours sensibles à des facteurs déstabilisants tels que la concentration en sels, la température, etc. ce qui n'est pas le cas des adjuvants de la présente invention. Leur stabilité correspond en principe à la stabilité des polymères contenant exclusivement des unités constitutionnelles répétitives anioniques, tels que les acides polyacryliques.

Un des avantages de l'adjuvant selon la présente invention est qu'il est efficace à faible dose. On peut donc augmenter la charge d'antigènes par volume injecté. Dans les vaccins à base de W/O, l'huile minérale occupe environ 50 % du volume du vaccin, tandis que la fraction volumique occupée par les adjuvants selon la présente invention (p. ex. à base d'acides polyacryliques liés à des chaînes d'hydrocarbures) peut être diminuée jusqu'à environ 10 % du volume de vaccin.

Selon un premier mode de réalisation avantageux, le poids moléculaire des polymères est compris entre 10 kD et 10.000 kD.

Avantageusement, le rapport molaire des unités répétitives constitutionnelles hydrophobes et des unités répétitives constitutionnelles anioniques est compris entre 0,05 et 1,00 et, de préférence entre 0,10 et 0,40.

De préférence, la solubilité des polymères dans l'eau s'élève à au moins 1 g/litre.

Selon un autre aspect de la présente invention, un procédé pour obtenir le polymère est décrit. Le polymère peut être obtenu par un des procédés suivants :

    1. copolymérisation de monomères anioniques et hydrophobes,
    2. greffage partiel de polymères,
    3. hydrolyse partielle de polymères, et
    4. par un anhydride intermédiaire.

Selon un autre mode de réalisation préféré, il est proposé un vaccin dont la concentration du polymère est comprise entre 1 et 40 mg/ml de vaccin, de préférence entre 4 et 24 mg/ml de vaccin, et encore de préférence entre 8 et 16 mg/ml de vaccin.

Selon un autre aspect de la présente invention le vaccin comprend des antigènes inactivés du virus de la maladie de Newcastle (NDV) et/ou du virus de la bronchite infectieuse (IBV) pour la vaccination d'animaux domestiques.

Les vaccins comprenant un adjuvant à base d'acides polyacryliques liés à des chaînes d'hydrocarbures sont beaucoup plus stables que les vaccins comprenant un adjuvant à base d'une émulsion W/O ou O/W , ou à base d'eau dans l'huile minérale dans l'eau (W/O/W) car l'adjuvant est une solution.

Selon encore un autre aspect de la présente invention, on propose l'utilisation d'une solution aqueuse de polymères ayant des unités constitutionnelles répétitives anioniques et des unités constitutionnelles répétitives hydrophobes en tant qu'adjuvant dans des vaccins.

On prévoit, selon encore un autre aspect de la présente invention, un procédé pour préparer un vaccin en solution, caractérisé en ce qu'on mélange une solution aqueuse d'un antigène et un polymère ayant des unités constitutionnelles répétitives anioniques et des unités constitutionnelles répétitives hydrophobes.

Exemple 1

Différents polymères hydrosolubles selon l'invention ont été synthétisés par estérification partielle d'un acide polyacrylique d'une masse moléculaire de 450.000 D [Carbopol 907 (PAA), Goodrich, Cleveland, Ohio, U.S.A.]. Dans cette demande le terme "PAA" se réfère à "Carbopol 907". Cet acide polyacrylique a été estérifié avec différents hydroxyalcanes selon la méthode décrite par Cohen, H.L. dans J. Poly. Sci. 14, pp. 7-22 (1976). Les polymères résultant, appelés ci-après "alkyl-PAA", (cf. tableau 1) contiennent des unités monomériques de type acide acrylique et des unités monomériques de type acrylate d'alkyle.

Un gramme de PAA a été mis en solution dans 50 ml de l'alcanol correspondant et la solution a été chauffée jusqu'à 135 °C. Cinquante µl de $H_2SO_4$ ont été ajoutés et le mélange de réaction a été maintenu à 135 °C. La réaction a été arrêtée par refroidissement rapide du mélange de réaction et en ajoutant un volume d'eau distillée. Ensuite, le pH de la solution a été ajusté à pH = 6 et les solvants ont été évaporés à 80 °C à pression réduite ($10^{-6}$ bar). Les produits ainsi obtenus ont été mis en solution dans de l'eau distillee, dialysés contre de l'eau distillée et puis lyophilisés.

Les composés suivants, dont les propriétés principales sont reprises dans le tableau 1, ont été synthétisés : décyl-PAA (C10-PAA), octyl-PAA (C8-PAA), butyl-PAA (C4-PAA), et méthyl-PAA (C1-PAA).

Le degré d'estérification de ces composés a été déterminé par analyse RMN. Il est exprimé en % molaire.

Les alkyl-PAA ont été mis en solution dans un tampon phosphate (pH = 7,5), en chauffant légèrement si nécessaire, et un volume de la solution d'adjuvant a été mélangé avec un volume de la solution contenant l'antigène.

Tableau 1

| Propriétés principales des alkyl-PAA synthétisés | | | | |
|---|---|---|---|---|
| Produit | MW PAA (kD) | Longueur de la chaîne | % ester (molaire) | Rapport molaire (hydrophobe/anionique) |
| C8-PAA | 450 | C8 | 27 | 0,37 |
| C8-PAA | 450 | C8 | 12 | 0,14 |
| C8-PAA | 450 | C8 | 16 | 0,19 |
| C8-PAA | 450 | C8 | 16 | 0,19 |
| C4-PAA | 450 | C4 | 16 | 0,19 |
| C10-PAA | 450 | C10 | NT | NT |
| C8-PAA | 450 | C8 | 48 | 0,92 |
| C4-PAA | 450 | C4 tertiaire | NT | NT |
| C1-PAA | 450 | C1 | 15 | 0,18 |
| NT = non mesuré (not tested) | | | | |

Exemple 2

Des poules ont été immunisées par injection intramusculaire (IM) avec 0,5 ml de vaccin comprenant le virus NDV (Newcastle Disease Virus) inactivé (souche Kimber) et le virus IBV (Infectious Bronchitis Virus) inactivé, comprenant les souches M41 et D274, (iNDV/iIBV) sans adjuvant, à quatre semaines d'âge, puis à sept semaines d'âge, avec les mêmes antigènes mais cette fois-ci avec un adjuvant.

Les animaux du groupe de contrôle négatif ont reçu une solution saline tamponnée avec du phosphate (PBS) au lieu de l'adjuvant et les animaux du groupe de contrôle positif ont été vaccinés avec le vaccin comprenant un adjuvant classique à base d'eau dans l'huile minérale.

Trois semaines après la deuxième vaccination, les poules ont été saignées et dans certaines expériences, des échantillons de sang ont été collectés une deuxième fois plusieurs semaines plus tard.

Les échantillons de sang ont été incubés à température ambiante et après deux heures, les caillots sanguins ont été éliminés, les cellules restantes ont été enlevées par centrifugation (10 min. à 2700 g) et les échantillons de sérum de chaque animaux ont été recueillis et stockés à - 20 °C jusqu'à leur utilisation.

Des plaques de microtitration à 96 puits ont été saturées avec du NDV purifié, inactivé et dilué dans une solution tamponnée, au carbonate (pH = 9,6; 0,1 M) pendant deux heures à 37 °C. Les plaques ont été saturées avec 5 % (poids/volume) de lait écrémé (SKM) dans un tampon carbonate pendant une nuit à 4 °C.

Le sérum des poules a été traité avec du kaolin en incubant un volume de sérum avec 4 volumes d'une suspension à 25 % (poids/volume) de kaolin dans un tampon de borate (ICN Biomedicals, Inc. Costa Mesa, U.S.A.), pendant 30 minutes. Le kaolin a ensuite été éliminé par centrifugation.

Le sérum des poules a été prédilué 100 fois dans du PBS contenant 1 (poids/volume) d'albumine de sérum bovin (PBS/BSA). Les échantillons de sérum ont été dilués en série deux fois dans la même solution dans des plaques à 96 puits et les plaques ont été incubées pendant une à deux heures à 37 °C. Des anti-IgG de poules, produits dans la chèvre et couplés à la peroxydase, dilués 1/1000 dans du PBS/SKM, ont été ajoutés et les plaques ont été incubées pendant 1 à 2 heures à 37 °C. La quantité de peroxydase dans les plaques a été quantifiée par ajout d'une solution de substrat ABTS + $H_2O_2$ (Kirkegaard & Perry Labs., U.S.A.) et mesure de l'absorbance à 405 nm effectuée par un multiscan Titertek.

Les titres d'anticorps sont exprimés sous forme de valeur 2-log du coefficient de régression du diagramme de densité optique par rapport au facteur réciproque de dilution.

Le titre d'anticorps a aussi été exprimé par les moyennes géométriques (valeur 2-log +/- SEM) et les valeurs antilog de ces moyennes ($2^{moyennes}$). L'activité de l'adjuvant a été exprimée en tant que pourcentage d'augmentation calculé comme suit :

$$\% \text{ augmentation} = [\text{antilog(échantillon)} - \text{antilog(contrôle négatif)}]/$$

$$[\text{antilog(contrôle positif)} - \text{antilog(contrôle négatif)}] * 100.$$

Des tests de Student ont été effectués pour analyser la signification statistique de ces résultats et la valeur P > 0,05 a été considérée comme étant significative.

Dans quatre expériences indépendantes, l'effet adjuvant d'un ou plusieurs acides polyacryliques partiellement estérifiés a été comparée à l'adjuvance de PAA non-estérifiés ainsi qu'avec un groupe de contrôle négatif sans adjuvant (PBS) et un groupe de contrôle positif avec de l'eau dans l'huile minérale (W/O).

Les titres d'anticorps ont été déterminés par la méthode ELISA indirecte, décrite ci-dessus. Les résultats de ces expériences sont repris dans le tableau 2.

Tableau 2 : Effets de différentes préparations d'alkyl-PAA sur la réponse immunologique contre NDV/IBV, mesurés par ELISA indirect sur des poules.

| Adjuvant [mg/ml] | n | Titre d'anticorps contre NDV | | | Adjuvance |
| --- | --- | --- | --- | --- | --- |
| | | moyenne 2-log | SEM | antilog | % augment. |
| Expérience I | | | | | |
| W/O | 40 | 9.6 | 0.7 | 776 | 100 |
| PBS | 40 | 5.6 | 1.0 | 49 | 0 |
| PAA [8] | 20 | 7.1 | 1.1 | 139 | 12 |
| PAA [40] | 20 | 6.8 | 1.0 | 110 | 8 |
| C8-PAA [8] | 20 | 9.0 | 0.6 | 491 | 61 |
| C8-PAA [40] | 20 | 9.3 | 0.8 | 630 | 80 |
| Expérience II | | | | | |
| W/O | 40 | 9.5 | 0.5 | 724 | 100 |
| PBS | 40 | 6.8 | 0.9 | 111 | 0 |
| PAA [8] | 20 | 7.4 | 1.0 | 162 | 8 |
| PAA [40] | 20 | 8.3 | 0.7 | 324 | 34 |
| C8-PAA [8] | 20 | 8.8 | 0.6 | 452 | 55 |
| C8-PAA [40] | 20 | 8.2 | 1.1 | 274 | 28 |
| C8-PAA [8] | 20 | 8.5 | 0.7 | 372 | 42 |
| C8-PAA [40] | 20 | 8.6 | 0.7 | 393 | 45 |
| C8-PAA [8] | 20 | 8.9 | 0.5 | 488 | 61 |
| C8-PAA [40] | 20 | 8.7 | 0.9 | 405 | 47 |

| Expérience III | | | | | |
|---|---|---|---|---|---|
| W/O | 40 | 8.9 | 0.9 | 477 | 100 |
| PBS | 40 | 5.7 | 0.6 | 52 | 0 |
| PAA [8] | 20 | 6.9 | 0.8 | 119 | 16 |
| PAA [24] | 20 | 7.3 | 0.7 | 158 | 25 |
| C8-PAA [8] | 20 | 8.4 | 0.7 | 338 | 69 |
| C8-PAA [8] | 20 | 8.3 | 0.7 | 315 | 64 |
| C8-PAA [24] | 20 | 8.3 | 0.5 | 315 | 64 |
| C8-PAA [8] | 20 | 9.1 | 0.3 | 549 | 121 |
| C8-PAA [8] | 20 | 8.4 | 0.5 | 338 | 69 |
| C8-PAA [24] | 20 | 8.0 | 0.6 | 256 | 49 |
| C4-PAA [8] | 20 | 8.8 | 0.6 | 446 | 96 |
| C4-PAA [24] | 20 | 8.6 | 0.5 | 388 | 81 |
| C10-PAA [8] | 20 | 7.1 | 0.7 | 137 | 20 |
| C10-PAA [24] | 20 | 5.8 | 0.6 | 56 | 0 |
| Expérience IV | | | | | |
| W/O | 40 | 8.6 | 1.0 | 388 | 100 |
| PBS | 40 | 5.2 | 1.1 | 37 | 0 |
| PAA [1] | 20 | 6.2 | 1.7 | 74 | 9 |
| PAA [2] | 20 | 6.0 | 1.0 | 64 | 6 |
| PAA [4] | 20 | 6.8 | 1.1 | 111 | 18 |
| PAA [8] | 20 | 7.2 | 1.0 | 147 | 26 |
| PAA [16] | 20 | 7.4 | 0.8 | 168 | 32 |
| C4-PAA [1] | 20 | 7.7 | 0.7 | 208 | 42 |
| C4-PAA [2] | 20 | 8.1 | 0.5 | 274 | 57 |
| C4-PAA [4] | 20 | 8.5 | 0.5 | 362 | 79 |

| C4-PAA [8] | 20 | 8.7 | 0.6 | 416 | 92 |
|---|---|---|---|---|---|
| C4-PAA [16] | 20 | 9.1 | 0.4 | 549 | 124 |
| C8-PAA [2] | 20 | 8.4 | 0.6 | 338 | 73 |
| C8-PAA [8] | 20 | 8.9 | 0.8 | 478 | 107 |
| C8-PAA [8] | 20 | 8.3 | 0.7 | 315 | 67 |
| *C4-PAA [8] | 20 | 7.3 | 0.9 | 158 | 29 |
| C1-PAA [8] | 20 | 8.2 | 0.7 | 294 | 62 |

n = nombre de poules par groupe SEM = déviation standard de la moyenne (Standard Error of Mean)

* = C4tertiaire

Les groupes de contrôle positif et négatif ont donné des titres d'anticorps reproductibles dans les quatre expériences indépendantes et les titres d'anticorps du contrôle positif ont été de trois à quatre unités 2-log supérieures (8 à 16 fois) à ceux des groupes de contrôle négatif.

Les PAA (non-modifiés) ont augmenté le titre en anticorps anti-iNDV entre 6 % et 32 % (Expérience I à IV) et il semble que cette augmentation dépende de la dose (Expérience IV). Une stimulation optimale est obtenue avec une dose de 40 mg/ml (Expérience II).

Les alkyl-PAA ont provoque des réponses significativement plus élevées que les PAA non-modifiés. L'octyl-PAA et le butyl-PAA produisaient des titres plus élevés que le décyl-PAA, le t-butyl-PAA ou le méthyl-PAA.

Des réponses maximales ont été obtenues avec des doses variant de 8 mg/ml à 40 mg/ml d'octyl-PAA ou de butyl-PAA. Les réponses ont été aussi élevées que les réponses obtenues avec un adjuvant classique à base d'huile et d'eau, utilisé en tant que contrôle positif.

L'expérience IV montre que la réponse dépend de la dose de PAA modifiés utilisée, du moins en ce qui concerne le butyl-PAA dans une plage allant de 1 mg/ml à 16 mg/ml. Même à dosages très faibles - de 1 mg/ml à 2 mg/ml -, le butyl-PAA augmentait la réponse humorale d'une manière significative. Des titres d'anticorps comparables à ceux rencontrés avec des doses beaucoup plus élevées de PAA non-modifiés ont été atteints.

La réponse humorale augmente avec la dose de PAA modifiés employée et atteint un maximum qui est équivalent à celle provoquée par les adjuvants classiques à base d'eau et huile. Des doses de 24 mg/ml et 40 mg/ml de butyl-PAA et d'octyl-PAA induisaient des réponses parfois plus faibles que des doses de 8 mg/ml, indiquant l'existence d'une concentration optimale pour ces composés, qui est comprise entre 8 et 24 mg/ml.

Exemple 3

Les titres d'anticorps anti-NDV dans les échantillons de sérum individuels ont également été déterminés par un kit ELISA anti-iNDV, disponible dans le commerce (Flockcheck Newcastle disease antibody test kit; IDEXX Labs, Inc. Maine, U.S.A.) selon le mode opératoire.

Les résultats de ces analyses sont repris dans le tableau 3.

Tableau 3 : Effet de différentes préparations de PAA sur le titre
d'anticorps anti-iNDV mesuré par un kit IDEXX ELISA sur des poules.

| Adjuvant [mg/ml] | n | Titre d'anticorps contre NDV | | | Adjuvance |
|---|---|---|---|---|---|
| | | moyenne 2-log | SEM | antilog | % augment. |
| Expérience I | | | | | |
| W/O | 40 | 14.5 | 0.7 | 23170 | 100 |
| PBS | 40 | 7.9 | 2.2 | 239 | 0 |
| PAA [8] | 20 | NT | | | |
| C8-PAA [8] | 20 | 13.7 | 0.8 | 13308 | 57 |
| C8-PAA [40] | 20 | 12.9 | 1.7 | 7643 | 32 |
| Expérience II | | | | | |
| W/O | 40 | 13.9 | 0.8 | 15268 | 100 |
| PBS | 40 | 8.7 | 1.9 | 416 | 0 |
| PAA [8] | 20 | 12.6 | 1.1 | 6209 | 39 |
| PAA [40] | 20 | NT | | | |
| C8-PAA [8] | 20 | 12.4 | 1.1 | 5404 | 36 |
| C8-PAA [40] | 20 | NT | | | |
| C8-PAA [8] | 20 | 12.3 | 1.5 | 5042 | 31 |
| C8-PAA [40] | 20 | 12.4 | 1.2 | 5404 | 36 |

| | | | | | |
|---|---|---|---|---|---|
| C8-PAA [8] | 20 | 12.5 | 1.0 | 5793 | 36 |
| C8-PAA [40] | 20 | 12.0 | 1.9 | 4096 | 25 |
| Expérience III | | | | | |
| W/O | 40 | 13.1 | 1.3 | 8780 | 100 |
| PBS | 40 | 8.2 | 1.8 | 294 | 0 |
| PAA [8] | 20 | NT | | | |
| PAA [24] | 20 | NT | | | |
| C8-PAA [8] | 20 | 13.5 | 1.0 | 11585 | 133 |
| C8-PAA [8] | 20 | 12.8 | 1.1 | 7132 | 81 |
| C8-PAA [24] | 20 | 13.5 | 1.3 | 11585 | 133 |
| C8-PAA [8] | 20 | 13.7 | 0.6 | 13308 | 153 |
| C8-PAA [8] | 20 | 12.5 | 1.1 | 5793 | 65 |
| C8-PAA [24] | 20 | NT | | | |
| C4-PAA [8] | 20 | 13.7 | 1.2 | 13308 | 158 |
| C4-PAA [24] | 20 | 14.2 | 0.8 | 18820 | 218 |
| C10-PAA [8] | 20 | NT | | | |
| C10-PAA [24] | 20 | NT | | | |
| Expérience IV | | | | | |
| W/O | 40 | 12.9 | 1.6 | 7643 | 100 |
| PBS | 40 | 7.5 | 2.3 | 181 | 0 |
| PAA [1] | 20 | NT | | | |
| PAA [2] | 20 | NT | | | |
| PAA [4] | 20 | NT | | | |
| PAA [8] | 20 | NT | | | |
| PAA [16] | 20 | NT | | | |
| C4-PAA [1] | 20 | NT | | | |

| | | | | | |
|---|---|---|---|---|---|
| C4-PAA [2] | 20 | NT | | | |
| C4-PAA [4] | 20 | 13.4 | 1.0 | 10809 | 142 |
| C4-PAA [8] | 20 | 13.4 | 1.0 | 10809 | 142 |
| C4-PAA [16] | 20 | 13.8 | 0.9 | 14263 | 189 |
| C8-PAA [2] | 20 | NT | | | |
| C8-PAA [8] | 20 | 13.2 | 0.9 | 9410 | 124 |
| C8-PAA [8] | 20 | 12.0 | 1.6 | 4096 | 52 |
| *C4-PAA [8] | 20 | NT | | | |
| C1-PAA [8] | 20 | 12.2 | 1.4 | 4705 | 60 |

n    = nombre de poules par groupe SEM   = déviation standard de la moyenne (Standard Error of Mean)

NT    = non mesuré (not tested)

*       = C4tertiaire

Dans les quatre expériences, les différences des valeurs moyennes des contrôles négatifs ont été faibles. Dans les quatre expériences, les différences des valeurs moyennes des contrôles positifs ont également été faibles. L'adjuvance des PAA modifiés se situe entre 23 et 218 %, le butyl-PAA et l'octyl-PAA ont été plus efficaces que le méthyl-PAA ou le décyl-PAA.

Les résultats obtenus par cette méthode confirment ceux de la première méthode d'analyse, développée spécialement pour ces tests.

Exemple 4

Dans des plaques à 96 puits, des dilutions progressives de l'anti-sérum ont été incubées avec $10^6$ particules infectieuses (PFU) d'une souche NDV Kimber pendant 18 heures à 37 °C. A chaque puits, $10^5$ cellules de la lignée aviaire QT35 ont été ajoutées et mises en plaque. Ces plaques ont été couvertes et incubées pendant 48 heures supplémentaires à 37 °C. Des dilutions de sérum apportant 50 % de réduction du nombre de particules infectieuses ont été considérées comme étant le titre d'anticorps.

Les résultats de ces expériences sont repris dans le tableau 4.

Tableau 4 : Effets de différentes préparations de PAA sur le titre d'anticorps anti-iNDV mesurés par neutralisation de virus (VN) sur des poules.

| Adjuvant [mg/ml] | n | Titre d'anticorps contre NDV | | | Adjuvance |
| | | moyenne 2-log | SEM | antilog | % augment. |
|---|---|---|---|---|---|
| Expérience I | | | | | |
| W/O | 40 | 16.3 | 2.1 | 80684 | 100 |
| PBS | 40 | 8.1 | 1.8 | 274 | 0 |
| PAA [8] | 20 | 11.6 | 2.0 | 3104 | 4 |
| C8-PAA [8] | 20 | 15.1 | 2.4 | 35120 | 43 |
| C8-PAA [40] | 20 | 14.0 | 2.3 | 16384 | 20 |
| Expérience II | | | | | |
| W/O | 40 | 16.0 | 2.0 | 65536 | 100 |
| PBS | 40 | 11.1 | 2.0 | 2194 | 0 |
| PAA [8] | 20 | 11.5 | 1.8 | 2896 | 1 |
| PAA [40] | 20 | 13.0 | 2.2 | 8192 | 9 |
| C8-PAA [8] | 20 | 15.2 | 1.8 | 37641 | 56 |
| C8-PAA [40] | 20 | 11.4 | 2.9 | 2702 | 1 |
| C8-PAA [8] | 20 | 13.8 | 2.2 | 14263 | 19 |
| C8-PAA [40] | 20 | 14.7 | 2.0 | 26616 | 38 |
| C8-PAA [8] | 20 | 15.6 | 1.6 | 49667 | 75 |
| C8-PAA [40] | 20 | 13.7 | 3.0 | 13308 | 18 |
| Expérience III | | | | | |
| W/O | 40 | 16.2 | 1.0 | 75281 | 100 |
| PBS | 40 | 10.4 | 1.9 | 1351 | 0 |
| PAA [8] | 20 | 12.6 | 2.2 | 6208 | 8 |
| PAA [24] | 20 | 14.8 | 1.2 | 28526 | 37 |
| C8-PAA [8] | 20 | 15.4 | 1.7 | 43237 | 57 |
| C8-PAA [8] | 20 | 14.6 | 1.4 | 24834 | 32 |

| | | | | | |
|---|---|---|---|---|---|
| C8-PAA [24] | 20 | 15.9 | 0.8 | 61147 | 81 |
| C8-PAA [8] | 20 | 16.0 | 0.6 | 65536 | 87 |
| C8-PAA [8] | 20 | 15.5 | 1.4 | 46341 | 61 |
| C8-PAA [24] | 20 | 15.9 | 0.8 | 61147 | 81 |
| C4-PAA [8] | 20 | 16.1 | 0.6 | 70240 | 93 |
| C4-PAA [24] | 20 | 16.2 | 2.4 | 75281 | 100 |
| C10-PAA [8] | 20 | 13.3 | 1.7 | 10086 | 12 |
| C10-PAA [24] | 20 | 12.1 | 1.5 | 43900 | 58 |
| Expérience IV | | | | | |
| W/O | 40 | 15.4 | 2.0 | 43238 | 100 |
| PBS | 40 | 9.3 | 1.2 | 630 | 0 |
| PAA [1] | 20 | 10.9 | 2.8 | 1911 | 3 |
| PAA [2] | 20 | 10.2 | 1.1 | 1176 | 1 |
| PAA [4] | 20 | 11.0 | 1.3 | 2048 | 3 |
| PAA [8] | 20 | 12.1 | 1.6 | 4390 | 9 |
| PAA [16] | 20 | 12.6 | 1.3 | 6208 | 13 |
| C4-PAA [1] | 20 | 12.5 | 1.6 | 5793 | 12 |
| C4-PAA [2] | 20 | 14.1 | 1.1 | 17560 | 40 |
| C4-PAA [4] | 20 | 14.9 | 1.2 | 30574 | 70 |
| C4-PAA [8] | 20 | 14.7 | 1.2 | 26616 | 61 |
| C4-PAA [16] | 20 | 15.6 | 1.4 | 49667 | 115 |
| C8-PAA [2] | 20 | 14.3 | 1.1 | 20171 | 46 |
| C8-PAA [8] | 20 | 15.7 | 1.5 | 53232 | 123 |
| C8-PAA [8] | 20 | 14.0 | 1.2 | 16384 | 37 |
| *C4-PAA [8] | 20 | 12.0 | 1.3 | 4096 | 8 |
| C1-PAA [8] | 20 | 13.4 | 1.9 | 10809 | 24 |

n    = nombre de poules par groupe SEM  = déviation standard de la moyenne (Standard Error of Mean)

\*    = C4tertiaire

Les animaux des groupes de contrôle négatif et positif donnaient des titres d'anticorps reproductibles dans les quatre expériences indépendantes. Le pourcentage d'augmentation réalisé par le PAA non modifié se situait entre 4 à 37 % en fonction de la dose de PAA utilisée.

Tous les alkyl-PAA induisaient des réponses plus élevées que les PAA non modifiés.

La qualité des réponses induites par les alkyl-PAA dépendait du type de chaîne alkyle utilisée et du degré d'estérification du PAA.

Pour le butyl-PAA, on a observé une relation étroite entre la dose d'adjuvants et la réponse humorale (expérience IV) tandis que pour l'octyl-PAA, les réponses à une dose de 40 mg/ml ne provoquait pas toujours une réponse plus élevée que des doses plus faibles, indiquant que l'optimum de la quantité d'octyl-PAA se situerait entre 24 mg/ml et 40 mg/ml.

L'évaluation de la fonction biologique des anticorps effectuée par le test de neutralisation de virus (VN) montrait qu'il existe une corrélation étroite entre les deux tests ELISA et le test VN.

## Exemple 5

L'adjuvance des PAA a aussi été testée sur des souris.

Des groupes de six souris ont été vaccinés avec 25 µl d'un vaccin comprenant 1 volume d'une solution d'antigènes composée de 10 µg de virus influenza inactivé (souche MRC-11) et 1 mg d'ovalbumine (OVA) (SIGMA, U.S.A.) par ml et un volume d'adjuvant. Trois semaines après l'injection, les titres d'anticorps ont été déterminés par la méthode indirecte ELISA décrite à l'Exemple 1.

Le sérum de souris a été prédilué dans une solution à 5 % de lait écrémé dans du PBS (PBS/SKM). Les échantillons de sérum ont été dilués en série deux fois dans la même solution dans des plaques à 96 puits et les plaques ont été incubées pendant une à deux heures à 37 °C. Des anti-IgG de souris, produits dans la chèvre et couplés à une peroxydase, dilués 1/300 dans PBS/BSA, ont été ajoutés et les plaques ont été incubées pendant 1 à 2 heures à 37 °C. La quantité de peroxydase dans les plaques a été quantifiée par une solution substrat de ABTS + $H_2O_2$ (Kirkegaard & Perry Labs., U.S.A.) et la mesure de l'absorbance à 405 nm a été effectuée par un multiscan Titertek.

Les résultats de ces expériences sont repris dans le Tableau 5.

Tableau 5

| Adjuvance des alkyl-PAA sur des souris | | | | | |
|---|---|---|---|---|---|
| Adjuvant [mg/ml] | n | 2-log du titre d'anticorps contre | | | |
| | | MRC11 | | OVA | |
| | | moyenne | SEM | moyenne | SEM |
| Expérience I | | | | | |
| PAA [4.0] | 6 | 11.2 | 0.5 | 9.0 | 0.6 |
| C8-PAA [4.0] | 6 | 14.8 | 0.3 | 10.1 | 0.4 |
| C8-PAA [4.0] | 6 | 14.5 | 0.8 | 9.5 | 0.6 |
| C8-PAA [4.0] | 6 | 13.6 | 1.1 | 8.7 | 0.8 |
| C4-PAA [4.0] | 6 | 14.3 | 0.4 | 9.4 | 1.1 |
| - | 6 | 10.0 | 0.4 | 4.2 | 0.9 |
| Expérience II | | | | | |
| PAA [4] | 6 | 15.0 | 0.5 | 8.8 | 0.8 |
| PAA [2] | 6 | 14.2 | 0.6 | 8.0 | 0.6 |
| C8-PAA [4] | 6 | 14.9 | 0.4 | 8.7 | 0.7 |
| C8-PAA [2] | 6 | 14.6 | 0.9 | 8.8 | 0.5 |
| C4-PAA [4] | 6 | 15.5 | 0.2 | 9.5 | 0.7 |
| C4-PAA [2] | 6 | 15.2 | 0.4 | 9.7 | 0.3 |
| n = nombre de poules par groupe<br>SEM = déviation standard de la moyenne (Standard Error of Mean) | | | | | |

L'accroissement de l'adjuvance des PAA par l'addition des chaînes aliphatiques n'a été confirmé que partiellement sur des souris, car les deux expériences indépendantes montraient des effets différents.

Exemple 6

Outre l'adjuvance, d'autres propriétés sont importantes pour l'évaluation d'un vaccin. Entre autres, la réaction locale est un aspect important, bien qu'une certaine réaction à l'endroit d'injection soit acceptée en général par certaines espèces d'animaux. La toxicité locale a été testée in vivo en suivant le gonflement de la patte des souris après l'injection du vaccin dans le coussinet de la patte de la souris. Il a été montré que cette méthode est très sensible.

On a injecté (sous-cutané), dans la plante du pied arrière gauche de groupes de six souris, 25 μl +/- 5 μl d'un vaccin contenant 1 volume d'un adjuvant dilué avec un volume d'une solution d'antigène contenant 10 μg MRC-11 et 1 mg d'ovalbumine par ml de NaCl 0.9 % (p/v).

L'épaisseur de la patte a été mesurée un jour avant l'injection et à différents intervalles après l'injection par un appareil semi-électronique spécialement mis au point pour ce propos par l'Université d'Etat d'Utrecht aux Pays Bas. On a montré que la précision de cet appareil a été jusqu'à 0,02 mm près.

Le gonflement de la patte a été calculé en soustrayant l'épaisseur avant et après le traitement et est exprimé en tant que 0,01 mm.

Les résultats de ces expériences sont repris dans le tableau 6.

Tableau 6 : Réactogenicité de PAA et d'alkyl-PAA sur des souris.

Expérience I

| Adjuvant [mg/ml] | gonflement moyen ($10^{-2}$ mm) | | | | | |
|---|---|---|---|---|---|---|
| | Jours | | | | | |
| | 1 | 2 | 6 | 10 | 17 | 22 |
| PAA [4] | 27 | 63 | 96 | 47 | 31 | 29 |
| C8-PAA [4] | 82 | 113 | 174 | 108 | 71 | 46 |
| C8-PAA [4] | 185 | 186 | 197 | 126 | 91 | 49 |
| C8-PAA [4] | 181 | 191 | 165 | 82 | 58 | 38 |
| C4-PAA [4] | 76 | 111 | 222 | 138 | 85 | 72 |
| C8-PAA [4] | 23 | 17 | 33 | 8 | 16 | 7 |
| PBS | 2 | 0 | 0 | 0 | 0 | 0 |

Expérience II

| Adjuvant [mg/ml] | gonflement moyen ($10^{-2}$ mm) | | | | | | | |
|---|---|---|---|---|---|---|---|---|
| | Jours | | | | | | | |
| | 1 | 2 | 3 | 7 | 12 | 20 | 26 | 35 |
| PAA [4] | 37 | 73 | 91 | 68 | 51 | 40 | 38 | 42 |
| PAA [2] | 33 | 52 | 36 | 37 | 30 | 19 | 14 | 14 |
| C8-PAA [4] | 171 | 154 | 157 | 107 | 70 | 49 | 35 | 34 |
| C8-PAA [2] | 125 | 79 | 124 | 70 | 36 | 28 | 19 | 29 |
| C4-PAA [4] | 72 | 77 | 135 | 120 | 88 | 68 | 59 | 61 |
| C4-PAA [2] | 105 | 80 | 74 | 84 | 62 | 38 | 42 | 69 |
| PBS | 3 | 0 | 0 | 0 | 0 | 0 | 0 | 0 |

La toxicité locale des PAA sur des souris est modérée, le gonflement de la patte des souris atteint un maximum après quelques jours et décline ensuite graduellement au bout de deux ou trois semaines.

L'addition de chaînes alkyles sur les PAA augmente la réaction locale qui disparait cependant au bout de deux à cinq semaines selon la quantité injectée. Les groupes octyles incitent une réaction plus forte que les groupes butyles.

Par contre, le gonflement provoqué par l'injection d'un adjuvant à base d'huile et d'eau provoque des gonflements beaucoup plus importants qui persistent pendant plus de huit semaines.

**Revendications**

1. Adjuvant pour vaccins comprenant une solution aqueuse d'un polymère ayant des unités constitutionnelles répétitives anioniques et des unités constitutionnelles répétitives hydrophobes.

2. Adjuvant selon la revendication 1, caractérisé en ce que les unités monomériques pour former les unités constitutionnelles répétitives anioniques sont choisies parmi les acides acrylique, méthacrylique, maléique, fumarique, éthylènesuifonique, vinylsulfurique, styrènesulfonique, vinylphénylsulfurique, 2-méthacryloyloxy éthanesulfonique, 3-méthacryloyloxy-2-hydroxypropanesulfonique, 2-acryl-2-méthylpropanesulfonique, 3-acrylamido-3-méthylbutanoïque, 3-méthacrylamido-3-méthylbutanoïque, vinylphosphorique, 4-vinylbenzoïque, 3-vinyloxypropane-1-sulfonique et N-vinylsuccimidique.

3. Adjuvant selon la revendication 2, caractérisé en ce que les unités monomériques pour former les unités constitutionnelles répétitives anioniques sont choisies parmi les acides acrylique, méthacrylique, maléique, fumarique, éthylènesulfonique, vinylsulfurique et styrènesulfonique.

4. Adjuvant selon la revendication 3, caractérisé en ce que les unités monomériques pour former les unités constitutionnelles répétitives anioniques sont choisies parmi les acides acrylique, méthacrylique, maléique et fumarique.

5. Adjuvant selon la revendication 4, caractérisé en ce que les unités monomériques pour former les unités constitutionnelles répétitives anioniques sont des unités d'acide acrylique.

6. Adjuvant selon la revendication 1, caractérisé en ce que les unités monomériques pour former les unités constitutionnelles répétitives hydrophobes sont choisies parmi les esters alkyliques, cycloalkyliques et hydroxyalkyliques des acides acrylique, méthacrylique, maiéique, fumarique, éthylenesulfonique, vinylsulfurique, styrènesulfonique, vinylphénylsulfurique, 3-méthacryloyloxy-2-hydroxypropanesulfonique, 2-méthacryloyloxy éthanesulfonique, 2-acryl-2-méthylpropanesulfonique 3-acrylamido-3-méthylbutanoïque, 3-méthacrylamido-3-méthylbutanoïque, vinylphosphorique, 4-vinylbenzoïque, 3-vinyloxypropane-1-sulfonique ou N-vinylsuccimidique et les éthers.

7. Adjuvant selon la revendication 6, caractérise en ce que les unités monomériques pour former les unités constitutionnelles répétitives hydrophobes sont choisies parmi les esters alkyliques des acides acrylique, méthacrylique, maléique, fumarique, éthylènesulfonique, vinylsulfurique ou styrènesulfonique.

8. Adjuvant selon la revendication 7, caractérisé en ce que les unités monomériques pour former les unités constitutionnelles répétitives hydrophobes sont choisies parmi les esters alkyliques des acides acrylique, méthacrylique, maléique ou fumarique dont le groupe alkyle contient de 4 à 8 atomes de carbone.

9. Adjuvant selon la revendication 8, caractérisé en ce que les unités monomériques pour former les unités constitutionnelles répétitives hydrophobes sont choisies parmi les esters alkyliques linéaires d'acide acrylique dont le groupe alkyle contient de 4 à 8 atomes de carbone.

10. Adjuvant selon l'une quelconque des revendications 1 à 9, caractérisé en ce que le rapport molaire des unités répétitives constitutionnelles hydrophobes et des unités répétitives constitutionnelles anioniques est compris entre 0,05 et 1,00.

11. Adjuvant selon la revendication 1 à 10, caractérisé en ce que le rapport molaire des unités répétitives constitutionnelles hydrophobes et des unités répétitives constitutionnelles anioniques est compris entre 0,10 et 0,40.

12. Adjuvant selon la revendication 1, caractérisé en ce que le poids moléculaire des polymères est compris entre 10 kD et 10.000 kD.

13. Vaccin comprenant une quantité immunogénique d'un antigène et un adjuvant selon l'une quelconque des revendications précédentes.

14. Vaccin selon la revendication 13, caractérisé en ce que la concentration d'adjuvant est comprise entre 1 et 40 mg/ml, de préférence entre 4 et 24 mg/ml.

15. Vaccin selon la revendication 13, caractérisé en ce que, outre l'adjuvant, il comprend des antigènes inactivés du vies de la maladie de Newcastle (NDV) et/ou du virus de la bronchite infectieuse (IBV) pour la vaccination d'animaux domestiques.

16. Utilisation d'un polymère ayant des unités répétitives constitutionnelles hydrophobes et des unités répétitives constitutionnelles anioniques en tant qu'adjuvant dans un vaccin.

17. Utilisation selon la revendication 16, caractérisé en ce que le polymère a des unités répétitives constitutionnelles hydrophobes selon l'une des revendications 6 à 9 et des unités répétitives constitutionnelles anioniques selon l'une des revendications 2 à 5.

18. Procédé pour préparer un vaccin en solution caractérisé en ce qu'on mélange une solution aqueuse d'un antigène et un polymère ayant des unités répétitives constitutionnelles hydrophobes et des unités répétitives constitutionnelles anioniques.

**Claims**

1. Vaccine adjuvant comprising an aqueous solution of a polymer having anionic repeating constituent units and hydrophobic repeating constituent units.

2. Adjuvant according to Claim 1, characterized in that the monomeric units for forming the anionic repeating constituent units are chosen from acrylic, methacrylic, maleic, fumaric, ethylenesulphonic, vinylsulphuric, styrenesulphonic, vinylphenylsulphuric, 2-methacryloyloxyethanesulphonic, 3-methacryloyloxy-2-hydroxypropanesulphonic, 2-acryl-2-methylpropanesulphonic, 3-acrylamido-3-methylbutanoic, 3-methacrylamido-3-methylbutanoic, vinylphosphoric, 4-vinylbenzoic, 3-vinyloxypropane-1-sulphonic and N-vinylsuccimide acids.

3. Adjuvant according to Claim 2, characterized in that the monomeric units for forming the anionic repeating constituent units are chosen from acrylic, methacrylic, maleic, fumaric, ethylenesulphonic, vinylsulphuric and styrenesulphonic acids.

4. Adjuvant according to Claim 3, characterized in that the monomeric units for forming the anionic repeating constituent units are chosen from acrylic, methacrylic, maleic and fumaric acids.

5. Adjuvant according to Claim 4, characterized in that the monomeric units for forming the anionic repeating constituent units are acrylic acid units.

6. Adjuvant according to Claim 1, characterized in that the monomeric units for forming the hydrophobic repeating constituent units are chosen from the alkyl, cycloalkyl and hydroxyalkyl esters of acrylic, methacrylic, maleic, fumaric, ethylenesulphonic, vinylsulphuric, styrenesulphonic, vinylphenylsulphuric, 3-methacryloyloxy-2-hydroxypropanesulphonic, 2-methacryloyloxyethanesulphonic, 2-acryl-2-methylpropanesulphonic, 3-acrylamido-3-methylbutanoic, 3-methacrylamido-3-methylbutanoic, vinylphosphoric, 4-vinylbenzoic, 3-vinyloxypropane-1-sulphonic or N-vinylsuccimide acids and the ethers.

7. Adjuvant according to Claim 6, characterized in that the monomeric units for forming the hydrophobic repeating constituent units are chosen from the alkyl esters of acrylic, methacrylic, maleic, fumaric, ethylenesulphonic, vinylsulphuric or styrenesulphonic acids.

8. Adjuvant according to Claim 7, characterized in that the monomeric units for forming the hydrophobic repeating constituent units are chosen from the alkyl esters of acrylic, methacrylic, maleic or fumaric acids whose alkyl group contains from 4 to 8 carbon atoms.

9. Adjuvant according to Claim 8, characterized in that the monomeric units for forming the hydrophobic repeating constituent units are chosen from the linear alkyl esters of acrylic acid whose alkyl group contains from 4 to 8 carbon atoms.

10. Adjuvant according to any one of Claims 1 to 9, characterized in that the molar ratio of the hydrophobic constituent repeating units to the anionic constituent repeating units is between 0.05 and 1.00.

**11.** Adjuvant according to Claims 1 to 10, characterized in that the molar ratio of the hydrophobic constituent repeating units to the anionic constituent repeating units is between 0.10 and 0.40.

**12.** Adjuvant according to Claim 1, characterized in that the molecular weight of the polymers is between 10 kD and 10,000 kD.

**13.** Vaccine comprising an immunogenic quantity of an antigen and an adjuvant according to any one of the preceding claims.

**14.** Vaccine according to Claim 13, characterized in that the concentration of adjuvant is between 1 and 40 mg/ml, preferably between 4 and 24 mg/ml.

**15.** Vaccine according to Claim 13, characterized in that, in addition to the adjuvant, it comprises inactivated antigens of the Newcastle disease virus (NDV) and/or of the infectious bronchitis virus (IBV) for the vaccination of domestic animals.

**16.** Use of a polymer having hydrophobic constituent repeating units and anionic constituent repeating units as adjuvant in a vaccine.

**17.** Use according to Claim 16, characterized in that the polymer has hydrophobic constituent repeating units according to one of Claims 6 to 9 and anionic constituent repeating units according to one of Claims 2 to 5.

**18.** Method of preparing a vaccine in solution, characterized in that an aqueous solution of an antigen and a polymer having hydrophobic constituent repeating units and anionic constituent repeating units are mixed.

**Patentansprüche**

**1.** Adjuvans für Impfstoffe umfassend eine wässerige Lösung eines Polymers, welches repetitive anionische Struktureinheiten und repetitive hydrophobe Struktureinheiten aufweist.

**2.** Adjuvans nach Anspruch 1, dadurch gekennzeichnet, dass die Monomereinheiten zur Bildung der repetitiven anionischen Struktureinheiten ausgewählt sind aus der Gruppe Acrylsäure, Methacrylsäure, Maleinsäure, Fumarsäure, Ethylensulfonsäure, Vinylschwefelsäure, Styrolsulfonsäure, Vinylphenylschwefelsäure, 2-Methacryloyloxy-ethansulfonsäure, 3-Methacryloyloxy-2-hydroxypropansulfonsäure, 2-Acryl-2-methylpropansulfonsäure, 3-Acrylamido-3-methylisobuttersäure, 3-methacrylamido-3-methylisobuttersäure, Vinylphosphorsäure, 4-Vinylbenzoesäure, 3-Vinyloxipropan-1-sulfonsäure und N-Vinylsuccimidsäure.

**3.** Adjuvans nach Anspruch 2, dadurch gekennzeichnet, dass die Monomereinheiten zur Bildung der repetitiven anionischen Struktureinheiten ausgewählt sind aus der Gruppe Acrylsäure, Methacrylsäure, Maleinsäure, Fumarsäure, Ethylensulfonsäure, Vinylschwefelsäure und Styrolsulfonsäure.

**4.** Adjuvans nach Anspruch 3, dadurch gekennzeichnet, dass die Monomereinheiten zur Bildung der repetitiven anionischen Struktureinheiten ausgewählt sind aus der Gruppe Acrylsäure, Methacrylsäure, Maleinsäure und Fumarsäure.

**5.** Adjuvans nach Anspruch 4, dadurch gekennzeichnet, dass die Monomereinheiten zur Bildung der repetitiven anionischen Struktureinheiten Einheiten der Acrylsäure sind.

**6.** Adjuvans nach Anspruch 1, dadurch gekennzeichnet, dass die Monomereinheiten zur Bildung der repetitiven hydrophoben Struktureinheiten ausgewählt sind aus der Gruppe Alkylester, Zykloalkylester und Hydroxyalkylester der Acrylsäure, Methacrylsäure, Maleinsäure, Fumarsäure, Ethylensulfonsäure, Vinylschwefelsäure, Styrolsulfonsäure, Vinylphenylschwefelsäure, 3-Methacryloyloxy-2-hydroxypropansulfonsäure, 2-Methacryloyloxy-ethansulfonsäure, 2-Acryl-2-methylpropansulfonsäure, 3-Acrylamido-3-methylisobuttersäure, 3-Methacrylamido-3-methylisobuttersäure, Vinylphosphorsäure, 4-Vinylbenzoesäure, 3-Vinyloxypropan-1-sulfonsäure oder N-Vinylsuccimidsäure und der Ether.

19

7. Adjuvans nach Anspruch 6, dadurch gekennzeichnet, dass die Monomereinheiten zur Bildung der repetitiven hydrophoben Struktureinheiten ausgewählt sind aus der Gruppe der Alkylester der Acrylsäure, Methacrylsäure, Maleinsäure, Fumarsäure, Ethylensulfonsäure, Vinylschwefelsäure oder Styrolsulfonsäure.

8. Adjuvans nach Anspruch 7, dadurch gekennzeichnet, dass die Monomereinheiten zur Bildung der repetitiven hydrophoben Struktureinheiten ausgewählt sind aus der Gruppe der Alkylester der Acrylsäure, Methacrylsäure, Maleinsäure oder Fumarsäure, wobei die Alkylgruppe zwischen 4 und 8 Kohlenstoffatome aufweist.

9. Adjuvans nach Anspruch 8, dadurch gekennzeichnet, dass die Monomereinheiten zur Bildung der repetitiven hydrophoben Struktureinheiten ausgewählt sind aus der Gruppe der linearen Alkylester der Acrylsäure, wobei die Alkylgruppe zwischen 4 und 8 Kohlenstoffatome aufweist.

10. Adjuvans nach einem der Ansprüche 1 bis 9, dadurch gekennzeichnet, dass das molare Verhältnis der repetitiven hydrophoben Struktureinheiten zu den repetitiven anionischen Struktureinheiten zwischen 0,05 und 1,00 beträgt.

11. Adjuvans nach einem der Ansprüche 1 bis 10, dadurch gekennzeichnet, dass das molare Verhältnis der repetitiven hydrophoben Struktureinheiten zu den repetitiven anionischen Struktureinheiten zwischen 0,10 und 0,40 beträgt.

12. Adjuvans nach Anspruch 1, dadurch gekennzeichnet, dass das Molekulargewicht der Polymere zwischen 10 kD und 10.000 kD beträgt.

13. Impfstoff aufweisend eine immunogene Menge eines Antigens und ein Adjuvans gemäß einem der vorhergehenden Ansprüche.

14. Impfstoff nach Anspruch 13, dadurch gekennzeichnet, dass die Konzentration des Adjuvans zwischen 1 und 40 mg/ml, insbesondere zwischen 4 und 24 mg/ml, beträgt.

15. Impfstoff nach Anspruch 13, dadurch gekennzeichnet, dass er neben dem Adjuvans weiters inaktivierte Antigene des Newcastle-Krankheit-Virus (NDV) und/oder des infektiösen Bronchitisvirus (IBV) zur Impfung von Haustieren aufweist.

16. Verwendung eines Polymers, welches repetitive hydrophobe Struktureinheiten und repetitive anionische Struktureinheiten aufweist, als Adjuvans in einem Impfstoff.

17. Verwendung nach Anspruch 16, dadurch gekennzeichnet, dass das Polymer repetitive hydrophobe Struktureinheiten gemäß einem der Ansprüche 6 bis 9 und repetitive anionische Struktureinheiten gemäß einem der Ansprüche 2 bis 5 aufweist.

18. Verfahren zur Herstellung eines Impfstoffes in Lösung dadurch gekennzeichnet, dass eine wässerige Lösung eines Antigens und eines Polymers, welches repetitive hydrophobe Struktureinheiten und repetitive anionische Struktureinheiten aufweist, vermischt wird.